# EUROPEAN PATENT SPECIFICATION

(11) **EP 4 525 728 B1**
(45) Date of publication and mention of the grant of the patent: **25.03.2026**
(21) Application number: 23727555.7
(22) Date of filing: 18.05.2023
(51) Int. Cl.: A61B 8/00, A61B 6/00, A61B 5/00

(54) **MULTI-MODALITY IMAGE VISUALIZATION FOR STROKE DETECTION**
MULTIMODALE BILDVISUALISIERUNG ZUR ERKENNUNG VON SCHLAGANFÄLLEN
VISUALISATION D'IMAGE À MODALITÉS MULTIPLES POUR DÉTECTION D'ACCIDENT VASCULAIRE CÉRÉBRAL

(30) Priority: 20.05.2022 US 202263344150 P
(43) Date of publication of application: 26.03.2025
(73) Proprietor: Koninklijke Philips N.V., 5656 AG Eindhoven (NL)
(72) Inventor: SUTTON, Jonathan Thomas, 5656 AG Eindhoven (NL); WEESE, Rolf Jürgen, 5656 AG Eindhoven (NL); ZHENG, Mingxin, 5656 AG Eindhoven (NL)
(74) Representative: Philips Intellectual Property & Standards
(86) International application number: PCT/EP2023/063412
(87) International publication number: WO 2023/222845

(56) References cited:
- WO-A1-2015/087203
- US-A1- 2015 223 779
- US-A1- 2016 317 129

## Description

This invention relates to medical diagnostic imaging systems and, in particular, to the combined use of ultrasound imaging and near infrared spectroscopy for stroke detection and diagnosis.

Stroke is one of the most debilitating disorders known to medicine. The blockage of the flow of blood to the brain, ischemic stroke, or the rupture of a cranial blood vessel, hemorrhagic stroke, can rapidly result in paralysis or death. Attempts to achieve recanalization of an occluded blood vessel through thrombolytic drug therapy such as treatment with tissue plasminogen activator (tPA) has been reported to cause symptomatic intracerebral hemorrhage in a number of cases. Advances in the diagnosis and treatment of this crippling affliction are the subject of continuing medical research.

US 2016/0317129 A1 discloses the following: A method and system for treating a target area of a patient, for example an area of the brain which includes an occlusion: employ an ultrasound imaging apparatus to produce an ultrasound image of a region of a subject; register the ultrasound image to a computed tomography (CT) image dataset; identify in the ultrasound image a location of a target area via a marker of the target area produced from the CT image dataset; verify the location of the target area with the ultrasound imaging apparatus; and provide sonothrombolysis treatment to the target area while monitoring the target area with the ultrasound imaging apparatus.

US Pat. 8,211,023 (Swan et al.) describes a diagnostic ultrasound system and method which enable a clinician to transcranially visualize a region of the cerebral vasculature where blood clots may be present. Either two dimensional or three-dimensional imaging may be employed. The imaging of the vasculature is preferably enhanced by the administration of microbubbles. If the flow conditions of the vasculature indicate the presence of a partial or complete occlusion from a blood clot, a focused or pencil beam is directed to the location of the blockage to break up the clot by the vibrations and/or rupturing of the microbubbles, a procedure known as sonothrombolysis. In some instances the ruptured microbubbles may also release an encapsulated thrombolytic drug. The patent also describes monitoring the cranial vasculature by ultrasonic imaging for changes which are indicative of the recurrence of an occlusion so that medical aid can be alerted to the recurrent condition.

Another imaging modality which is useful in stroke detection is near infrared spectroscopy, or NIRS. NIRS is based on the same physical principle as pulse oximetry, in which blood inside the body is illuminated at a near infrared wavelength of light which is sensitive to the coloration of oxygenated blood. The intensity of the returning light at a detection wavelength is a measure of the oxygenation of the blood which was illuminated. In a cranial NIRS system the patient is fitted with a headset carrying numerous emitter laser diodes and high-sensitivity photo-diodes which transmit light at infrared frequencies into the head of the patient and receive non-absorbed light reflected to the photo-diodes. At the wavelengths used, the infrared light is capable of penetrating the cerebral cortex for a few centimeters (e.g., 2-3 cm) and reflected light is received by light-sensitive detectors to sense the quantum of oxygenated or de-oxygenated blood at the locations of the illumination. The amount of oxygenated or de-oxygenated blood and their relative proportions at different locations in the cerebral cortex can then be assessed for possible perfusion or blood flow deficiencies.

In diagnosing and treating stroke it is important to be able to quickly determine that a stroke has occurred, its location in the brain, and the type of stroke so that the appropriate treatment can be rapidly commenced. As discussed above, applying the wrong treatment, such as tPA when the patient has suffered hemorrhagic stroke, can have grievous consequences. One way to increase the certainty of a diagnosis is to perform diagnoses with different diagnostic modalities, so that the results of one diagnosis can confirm or challenge the other. US Pat. Pub. No. 2019/0231316 (Sutton et al.) illustrates a multi-modality approach to diagnosing and treating myocardial infarction, in which the results of a digital ECG diagnosis are used to aid in locating a thrombus for ultrasonic imaging and subsequent sonothrombolytic treatment. It would correspondingly be desirable to combine two modalities, ultrasonic imaging and NIRS imaging, for stroke detection and follow-on treatment.

WO 2015/087203 A1 describes methods and systems for monitoring treatment of lesions.

In accordance with the principles of the present invention, a combined ultrasonic cranial imaging system and near infrared spectroscopy system is described for diagnosing stroke. Both the ultrasound system and the NIRS system produce their cranial analytical data in a common spatial coordinate system. This enables the data to be merged together in a common analytical framework such as spatially corresponding images or fused into a single 2D or 3D cerebral image map. Pathology revealed by the image data of one modality should spatially correlate with pathology identified by the other modality, enabling a clinician to confidently proceed with the appropriate plan of treatment. For example the detection of a possibly occluded vessel in an ultrasound image can be confirmed by a lack of oxygenated blood in the cranial region downstream of the suspected occlusion. Such a system may employ a common transducer/sensor headset that can be quickly applied to a patient for the production of data of both modalities in a common coordinate system and resulting rapid diagnosis and treatment.

A multimodality imaging system which can diagnose abnormalities of the brain such as indicia of the occurrence of stroke comprises a NIRS system adapted to acquire NIRS signals from the brain and produce NIRS image data using the acquired NIRS signals; an ultrasound imaging system adapted to acquire ultrasound signals from the brain and produce ultrasound image data using the acquired ultrasound signals; an image processor adapted to register the NIRS image data and the ultrasound image data to a common system of spatial coordinates; and an image display adapted to display spatially related NIRS image data and ultrasound image data.

A multimodality method of the present invention which can be used for diagnosing abnormalities of the brain such as indicia of the occurrence of stroke comprises operating a NIRS system adapted to acquire NIRS signals from the brain and produce NIRS image data using the acquired NIRS signals; operating an ultrasound imaging system adapted to acquire ultrasound signals from the brain and produce ultrasound image data using the acquired ultrasound signals; operating an image processor adapted to register the NIRS image data and the ultrasound image data to a common system of spatial coordinates; and operating an image display adapted to display spatially related NIRS image data and ultrasound image data.

A method in accordance with the present invention which can be used for diagnosing a suspect region of a body using multiple imaging modalities comprises acquiring image data of a region of the body using a first imaging modality; acquiring image data of the region of the body using a second imaging modality, wherein the image data of both imaging modalities is related to a common coordinate system; producing images of the region of the body using the image data of each modality; detecting regions of suspect pathology in the images of both modalities; merging the image data of the detected regions into a 3D dataset; selecting a region of suspect pathology; displaying an image of one or both modalities of the selected region of suspect pathology; and displaying diagnostic data related to the suspect pathology.

In the drawings:
FIGURE 1 illustrates in block diagram form an ultrasonic diagnostic imaging system constructed in accordance with the principles of the present invention.
FIGURES 2 illustrates a cranial headset for use with a handheld ultrasound transducer probe.
FIGURE 3 illustrates a headset for both NIRS emitters and sensors and ultrasound imaging probes.
FIGURE 4 illustrates a low-profile ultrasound transducer probe for an ultrasound/NIRS headset.
FIGURE 5 illustrates cranial ultrasound scanning by two contralateral transducer arrays.
FIGURE 6 illustrates a cranial ultrasound image.
FIGURE 7 illustrates a cranial NIRS image.
FIGURE 8 illustrates an image display which displays spatially corresponding NIRS data and ultrasound image data in accordance with the principles of the present invention.
FIGURE 9 is an illustration of the concept of combining spatially aligned ultrasound and NIRS image data in accordance with the principles of the present invention.
FIGURE 10 illustrates a 3D memory and volume rendering processor for production of a 3D cranial vascular map containing both ultrasound and NIRS image data in accordance with the principles of the present invention.
FIGURE 11a and 11b are a flowchart illustrating a method which can be used for multimodality diagnosis of a suspect region of the body using ultrasound imaging and NIRS imaging.

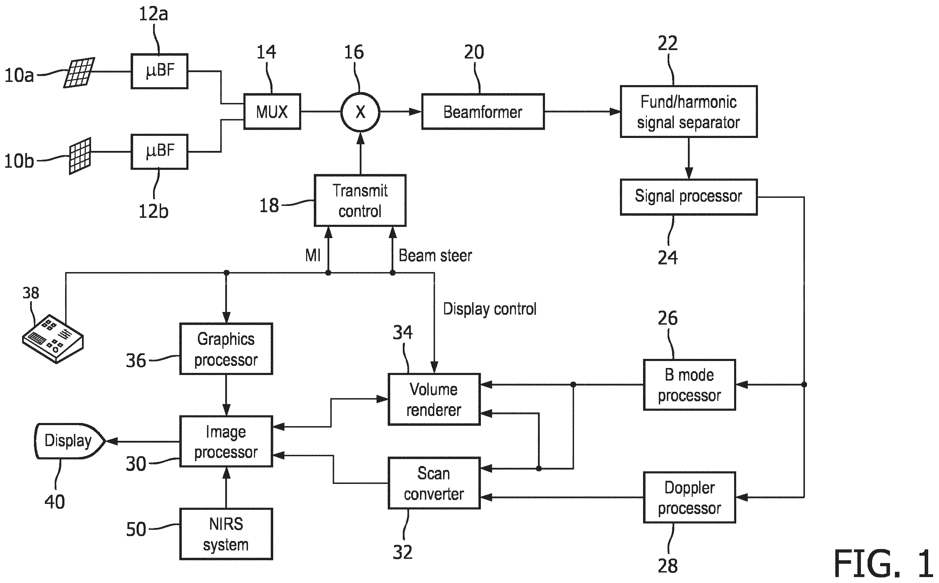

Referring first to FIGURE 1, a combined ultrasound and NIRS system constructed in accordance with the principles of the present invention is shown in block diagram form. Two transducer arrays 10a and 10b are provided for transmitting ultrasonic waves and receiving echo information. In this example the arrays shown are two dimensional arrays of transducer elements (matrix arrays) capable of scanning a volumetric region and providing 3D image data for imaging. The transducer arrays are coupled to microbeamformers 12a and 12b which control transmission and reception of signals by the array elements. Microbeamformers are also capable of at least partial beamforming of the signals received by groups or "patches" of transducer elements as described in US Pats. 5,997,479 (Savord et al.), 6,013,032 (Savord), and 6,623,432 (Powers et al.) Signals are routed to and from the microbeamformers by a multiplexer 14 by time-interleaving signals. The multiplexer is coupled to a transmit/receive (T/R) switch 16 which switches between transmission and reception and protects the main beamformer 20 from high energy transmit signals. The transmission of ultrasonic beams from the transducer arrays 10a and 10b under control of the microbeamformers 12a and 12b is directed by the transmit controller 18 coupled to the T/R switch, which receives input from the user's operation of the user interface or control panel 38 and controls the steering direction and focusing of beams to and from the array transducer in accordance with system control settings.

The partially beamformed signals produced by the microbeamformers 12a, 12b are coupled to a main beamformer 20 where partially beamformed signals from the individual patches of elements are combined into fully beamformed signals. For example, the main beamformer 20 may have 128 channels, each of which receives a partially beamformed signal from a patch of 12 transducer elements. In this way the signals received by over 1500 transducer elements of a two-dimensional array can contribute efficiently to a single beamformed signal.

The beamformed signals are coupled to a fundamental/harmonic signal separator 22. The separator 22 acts to separate linear and nonlinear signals so as to enable the identification of the strongly nonlinear echo signals returned from microbubbles. The separator 22 may operate in a variety of ways such as by bandpass filtering the received signals in fundamental frequency and harmonic frequency bands, or by a process known as pulse inversion harmonic separation. A suitable fundamental/harmonic signal separator is shown and described in international patent publication WO 2005/074805 (Bruce et al.) The separated signals are coupled to a signal processor 24 where they may undergo additional enhancement such as speckle removal, signal compounding, and noise elimination.

The processed signals are coupled to a B mode processor 26 and a Doppler processor 28. The B mode processor 26 employs amplitude detection for the imaging of structures in the body such as muscle, tissue, and blood vessels. B mode images of structures of the body may be formed in either the harmonic mode or the fundamental mode. Tissues in the body and microbubbles both return both types of signals and the harmonic returns of microbubbles enable microbubble-perfused tissue and blood flow to be clearly segmented in an image. The Doppler processor processes temporally distinct signals from moving tissue and blood flow for the detection of motion of substances in the image field including microbubbles. The structural and motion signals produced by these processors are coupled to a scan converter 32 and a volume renderer 34, which produce image data of tissue structure, flow, or a combined image of both characteristics. The scan converter will convert echo signals with polar coordinates into image signals of the desired image format such as a sector image in Cartesian coordinates. The volume renderer 34 will convert a 3D data set into a projected 3D image as viewed from a given reference point as described in US Pat. 6,530,885 (Entrekin et al.) As described therein, when the reference point of the rendering is changed the 3D image can appear to rotate or be viewed from a different direction in what is known as kinetic parallax. This image manipulation is controlled by the user as indicated by the Display Control line between the user interface 38 and the volume renderer 34. Also described is the representation of a 3D volume by planar images of different image planes, a technique known as multiplanar reformatting. The volume renderer 34 can operate on image data in either rectilinear or polar coordinates as described in US Pat. 6,723,050 (Dow et al.) The 2D or 3D images are coupled from the scan converter and volume renderer to an image processor 30 for further enhancement, buffering and temporary storage for display on an image display 40.

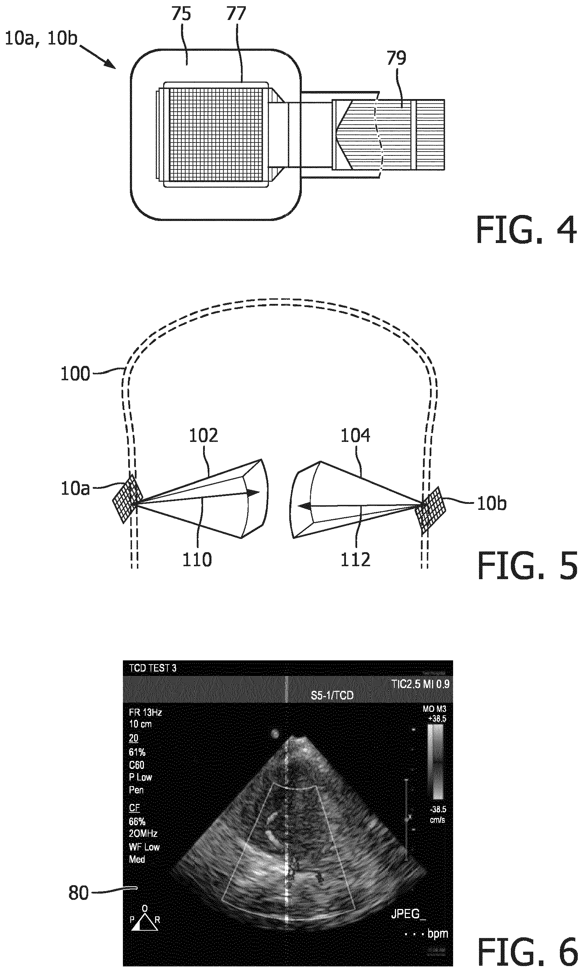

A graphics processor 36 is also coupled to the image processor 30 which generates graphic overlays for displaying with the ultrasound images. These graphic overlays can contain standard identifying information such as patient name, date and time of the image, imaging parameters such as depth and the like, and can also produce a graphic overlay of a beam vector steered by the user as shown in FIGURE 5. For this purpose the graphics processor receives input from the user interface 38. The user interface is also coupled to the transmit controller 18 to control the generation of ultrasound signals from the transducer arrays 10a and 10b and hence the images produced by and therapy applied by the transducer arrays. The transmit parameters controlled in response to user adjustment include the MI (Mechanical Index) which controls the peak pressure of the transmitted waves, which may be a low MI for harmonic imaging of microbubbles or a high MI for sonothrombolytic therapy, for instance. Other transmit parameters control the steering of the transmitted beams for image positioning and/or positioning (steering) of a therapy beam.

In accordance with the principles of the present invention, a near infrared spectroscopy (NIRS) system 50 is coupled to the image processor, enabling NIRS image data to be coupled to the system for viewing and combining with the ultrasound information. The NIRS system 50 can comprise a commercially available system such as the NIRScout system available from NIRx Medizintechnik GmbH of Berlin, Germany, or the Hitachi ETG 4100 fNIRS system available from Philips Healthcare of Eindhoven, The Netherlands. NIRS is based upon the strong chromophoric or light-absorbing properties of the hemoglobin molecule. The NIRS system transmits near infrared light into the skull at wavelengths which are absorbed by hemoglobin, and signals in the form of non-absorbed light is reflected back to one or more photodiodes where it is detected for production of an estimate of the amount of hemoglobin at the illuminated location. Since oxyhemoglobin and de-oxyhemoglobin absorb light differently, it is preferable to use two different wavelengths of light, 695nm-760nm and 830nm-900nm, so that the ratio between oxy- and deoxy-hemoglobin and cerebral arterial-venous oxygen saturation levels can be calculated. The data produced by a NIRS system is generally calibrated in arbitrary units, since it is derived from optical density units and converted mathematically to dimensionless units. Thus, NIRS data values are usually used comparatively, such as to monitor trends in which data values at a certain location may decline over time, or to compare differences in NIRS data values measured at different locations in the cerebral cortex. In healthy patients, the oxygen saturation in the cerebral cortex is generally uniform over the entire surface of the brain. Thus, a decreased oxyhemoglobin measurement at a specific region of the cortex is an indication of a possible problem. Since the brain is a physically symmetrical organ, comparative NIRS measurements are often made in corresponding locations of the left and right lobes of the cerebrum. Thus, measurements may be made at mirrored locations of the left and right lobes of the cerebrum and compared. In a normal patient, these two measurements should be very comparable. But if they differ significantly, such as by 20% or more, or a particular location shows a reduction in blood oxygenation of 20% or more compared to the rest of the brain, a possible oxygenated blood deficiency caused by an occlusion of a vessel feeding that area of the brain may be indicated. Comparative measurements such as these may be used in an implementation of the present invention. Accordingly, blood oxygen measurements made at specific locations in the cerebral cortex are coupled to image processor 30 of the ultrasound system where they are processed in conjunction with ultrasound data to produce combined ultrasound/NIRS information for the clinician as described more fully below.

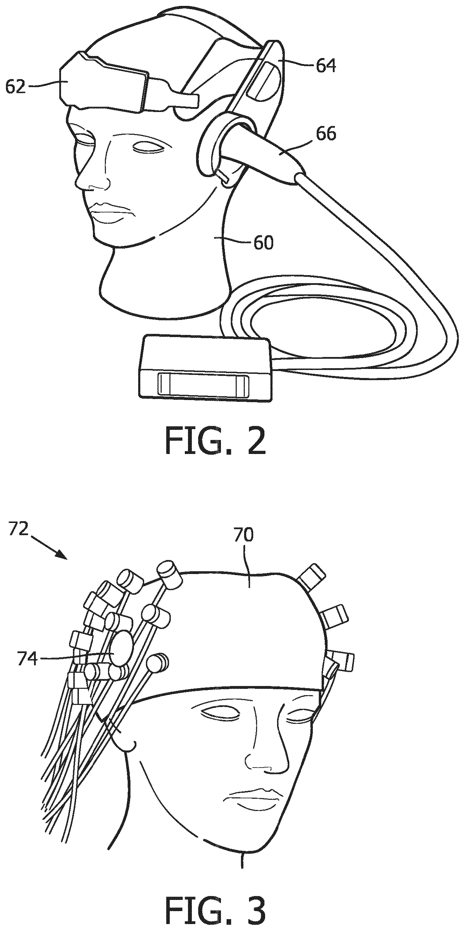

The transducer arrays 10a and 10b of the ultrasound system transmit ultrasonic waves into the cranium of a patient from opposite sides of the head, although other locations may also or alternately be employed such as the front of the head or the sub-occipital acoustic window at the back of the skull. The sides of the head of most patients advantageously provide suitable acoustic windows for transcranial ultrasound at the temporal bones around and above the ears on either side of the head. In order to transmit and receive echoes through these acoustic windows the transducer arrays must be in good acoustic contact at these locations which may be done by holding the transducer arrays against the head with a headset. For instance, FIGURE 2 shows a headset 62 for two matrix array probes 66 mounted on the head 60 of a mannequin. A headset may have a snap-on deformable acoustic standoff 64 which allows the transducer array to be manipulated by its conformal contact surface and aimed at the arteries within the brain while maintaining acoustic contact against the temporal window. The acoustic coupling objective is facilitated by integrating a mating spherical surface into the probe handle, which allows the probe to pivot in the headset 62 until it is strongly and tightly coupled to the temporal window of the patient.

FIGURE 3 illustrates a mannequin fitted with a NIRS cap 70 containing wired LED emitters and photodiode sensors 72 for illuminating and receiving reflected infrared light from the brain. The emitters and sensors are coupled to channels of the NIRS system for amplification and detection. The locations of the emitters and sensors in 3D space around the cap are known, so selected pairs can be activated and the regions of the cortex at which measurements are made can be identified by x,y,z coordinates. The NIRS cap 70 thus provides a framework for a common 3D coordinate system in which measurement locations in the head of the patient can be spatially identified and related to each other.

Also shown in FIGURE 3 is a white circle 74 which marks one of the locations on opposite sides of the head where an ultrasound transducer 10a, 10b may be located to access a cranial acoustic window. Since the location 74 of the acoustic window is at a known location of the cap in relation to the positions of the emitters and sensors 72, the ultrasound transducer location can thereby be identified in the same spatial coordinate system as the emitter and sensor locations. The NIRS cap 70 thus provides a common coordinate system for both NIRS and ultrasound measurements and images.

The use of a handheld ultrasound probe with the NIRS cap 70, such as the probe 66 in FIGURE 2, is often awkward and cumbersome due to the need to mount and support the probe with a head-mounted mechanism such as the headset 62. For a NIRS cap, it is preferable to use an ultrasound transducer 10a, 10b with a low-profile configuration that can be placed in or attached to the inside of the NIRS cap at a known location 74 (and an opposing location) and positioned in good acoustic contact with the head of the patient. FIGURE 4 illustrates a preferred ultrasound probe 10, 10b for an implementation of the present invention, which comprises a low-profile two-dimensional matrix array 77 of transducer elements. The matrix ultrasound transducer is formed as a patch that adheres to the patient's body with double-sided medical grade tape 75 so that it can remain in the same position inside the NIRS cap 70 during a procedure. A suitable matrix array patch is described in U.S. Pat. No. 6,685,647 (Savord et al.), which uses a de-matching layer for a low-profile assembly. The matrix array is formed as a standard piezoelectric-based acoustic stack connected through a ball grid or equivalent interconnect to a microbeamformer ASIC behind the array. FIGURE 4 shows a plan view of the face of the matrix array probe 10a, 10b. A flexible ribbon cable 79 couples the matrix array and microbeamformer to the ultrasound system. In use, the central matrix array area 77 of the probe is acoustically coupled to a patient's body in the area of interest with ultrasonic gel.

FIGURE 5 illustrates the volumetric image fields 102, 104 scanned by matrix array transducers 10a and 10b when acoustically coupled to the temples to scan through the skull 100. A clinician can image the cranial vasculature in these volumetric image fields and steer the pyramidal image fields in different directions to search for blood clots obstructing the cranial blood flow. At each position of the image field 102, 104 the clinician can look for obstructions of the blood flow in the real time images on the display, or can capture (freeze) an image or map of the cranial vasculature. When the vascular map is acquired and held statically, the image can undergo enhanced processing (e.g., compounding, signal averaging) to improve the resolution or scale of the image, and can be manipulated on the screen and examined carefully at different points and from different views in a precise search for blood vessel occlusions. In this way the clinician can diagnose for stenoses. If the clinician examines a vascular map and finds no evidence of obstruction in the blood flow paths, the clinician can steer the image field to another region of the cranium and examine the vascular map of another image field. The clinician can use the Doppler data of the vascular map or the spectral Doppler function of the ultrasound system to take flow velocity measurements at specific points in the cranial vasculature, indicated by cursors 110, 112 in the image, for comparative analysis with NIRS results.

Each volumetric ultrasound image field 102, 104 is seen to have an origin at a certain point on a matrix array 10a, 10b. Since each matrix array is positioned at a known location 74 of the NIRS cap, the result is that each point in the ultrasound image fields has 3D coordinates in the same spatial reference system as the NIRS emitters and sensors. Thus, the ultrasound image data and the NIRS image data can be spatially related to each other since they share a common spatial coordinate system for either 2D or 3D imaging.

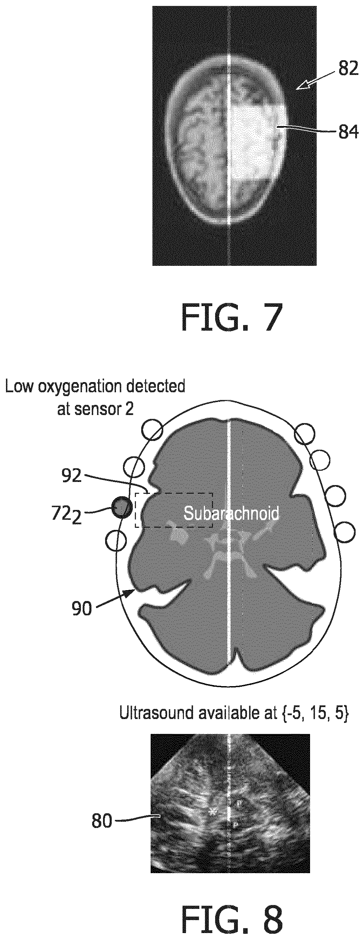

FIGURE 6 illustrates an ultrasound image 80 produced from ultrasound image data acquired by one of the transducers 10a, 10b. The brightly colored spots in the Doppler window of the image show the locations of cranial blood flow in the image field. An example of a display of NIRS image data is shown in FIGURE 7. For reference, the brighter NIRS image area 82 is shown in FIGURE 7 over an MRI image of a brain. The NIRS image is seen to contain darker spots 84 indicating a possible blood flow abnormality in the cortex on the right side of the image. A combined ultrasound/NIRS system of the present invention advantageously shows ultrasound and NIRS image data in a common reference framework as illustrated below.

FIGURE 8 shows a display produced by a multimodality system of the present invention. Prior to the production of an image display the image processor 30 accesses the geometrical locations (x,y, z and orientation data when present) of the NIRS sensors and ultrasound images (pixels or voxels) and processes it through a registration algorithm. Since both the NIRS and the ultrasound data were acquired in a common coordinate system, they can be registered to spatial coordinates which relate the data. At the top of the display is a head model template 90 which relates the NIRS and ultrasound data. Such head templates are available from open-source libraries such as the Colins27 Average Brain model from the McConnel Brain Imaging Centre. Alternatively, a CT or MRI image of the patient's head can be used as the template when available. The circles located around the brain model 90 mark the locations of the NIRS sensors such as Sensor 2, indicated at 72₂. The dashed box 92 to the right of Sensor 2 marks the location in the brain where NIRS data was acquired by Sensor 2 and a blood flow abnormality, low oxygenation, was detected. Below the head model template 90 is an ultrasound image 80 which was acquired from the region of the brain marked by the dashed box. Ultrasound image 80 can be related to the region of dashed box 92 and Sensor 2 since the NIRS sensors and ultrasound transducer and data are related to a common coordinate system as described above. Sensor circles around the head template such as circle 72₂ for which there is both NIRS and ultrasound data available are filled in with color; empty circles indicate a lack of one or both types of data for that brain location.

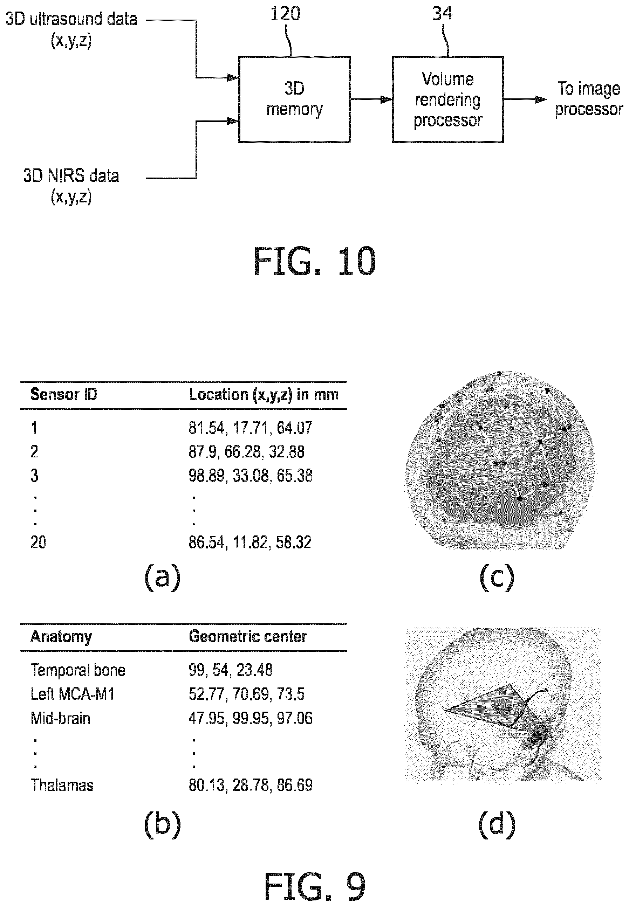

FIGURE 9 illustrates conceptually how NIRS data and ultrasound data can be merged to form co-registered images in a system of the present invention. With the known hardware configuration of the NIRS sensors 72 in the NIRS cap 70, one can obtain a table of the sensor locations relative to a model, such as that shown in FIGURE 9(a). The (x,y,z) coordinate locations of this table represent the arrangement of sensors 72 in the NIRS cap 70 as shown in FIGURE 9(c). The abstract locations of cerebral structures can be calculated by their geometrical centers in the NIRS data as illustrated in the table of FIGURE 9(b). After the locations of the NIRS sensors and cerebral structures are determined in the coordinate system of the NIRS cap, live dynamic ultrasound images can be registered to this coordinate system based on the detected features found in the ultrasound data. This approach can be used even when the ultrasound transducer location is not physically referenced to the NIRS cap, and freehand ultrasound scanning is performed. The registration can be done in an iterative method by searching all the optimal matches between 2D structures in the ultrasound image and their 3D locations in relation to the NIRS cap. Alternatively, linear regression may be applied to optimize the search process and provide the optimal localization of the ultrasound imaging plane or volume in relation to the NIRS sensors.

FIGURE 10 illustrates a hardware subsystem for combining 3D NIRS data and ultrasound data when the two data types are already referenced to a common coordinate system as by the NIRS cap of FIGURE 3. A 3D volume of NIRS data is stored in locations of a 3D memory 120 at locations addressed by the (x,y,z) coordinates. Similarly, a 3D volume of ultrasound data is stored in the memory 120 at locations addressed by its (x,y,z) coordinates. The data may be combined (e.g., summed) in the memory 120. The stored 3D data is then rendered into a 3D display by a volume rendering processor 34, which operates as described in the above-referenced Entrekin et al. patent. This is by processing data in virtual lines projected through the 3D data which are all in a desired look direction through the volume. The image data of both types which is encountered along a virtual line is processed by known techniques such as minimum or maximum intensity projection. A user control of the control panel 38 enables the operator to change or continuously vary the rendering parameter and the look direction, providing a dynamic parallax display that gives the impression of viewing a rotating or shifting volume.

Stroke regions and vascular structures in the NIRS and ultrasound data can be detected by advanced processing such as that performed by a neural network or deep learning software run on the image processor. The neural network is first trained with NIRS images and ultrasound images that contain clinically identified stroke regions and vascular structures. In the training images, regions to be detected have been annotated. Once properly trained with known images, the neural network or deep learning software can be used to segment diagnostically relevant regions in both types of images.

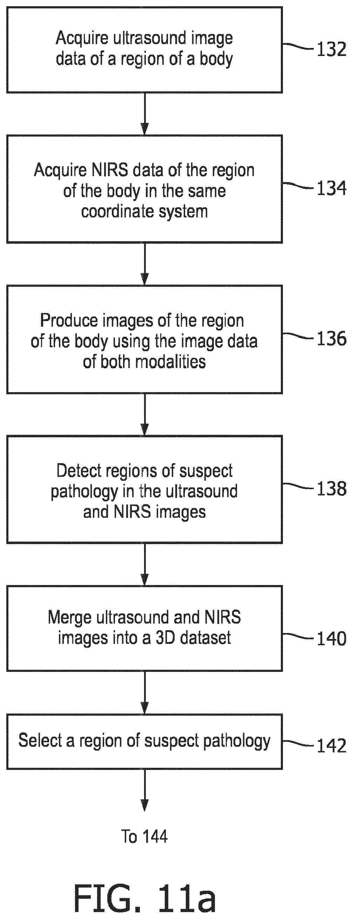

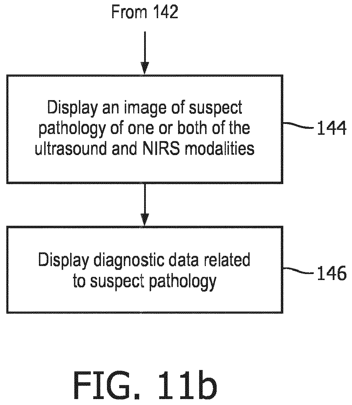

A method in accordance with the present invention which can be used for multimodality diagnosis of a suspect region of the body using ultrasound imaging and NIRS imaging is illustrated in FIGURES 11a and 11b. In step 132 ultrasound image data of a region of a body is acquired using one of more of the ultrasound probes 66. In step 134 NIRS data of the region of the body in the same coordinate system as the ultrasound data is acquired. As explained above, this may be done by affixing one or more ultrasound matrix array transducers 10a, 10b to a NIRS cap 70 which is then fitted to the head of a subject to acquire both ultrasound and NIRS data from the head of the patient. The NIRS cap thus supplies the same coordinate reference for both the ultrasound data and the NIRS data. In step 136 images of the region of the body are produced using the ultrasound data and the NIRS data. In step 138, one or more regions of suspect pathology are detected in the ultrasound and NIRS images. Detection can be done manually by the clinician who visually examines the images in detail and circles or highlights suspect regions in the images. This can be done using a vascular map of the anatomy as discussed above. Detection can also or alternatively be done automatically as described above by a neural network or deep learning software which has been trained to detect suspect pathology in images of the subject anatomy. In step 140, the ultrasound and NIRS images are merged into one 3D dataset as by the process illustrated in FIGURE 10. Alternately, the ultrasound and NIRS images can be shown separately, such as side-by-side or in alternation. In step 142 a region of suspect pathology is selected in the image data. This can be done manually by the clinician clicking on suspect pathology in an image with a pointing device such as a mouse or trackball or outlining the suspect pathology with the pointing device. An image of suspect pathology of one or both modalities is then displayed in step 144. Since the ultrasound and NIRS images are displayed in the common coordinate system, the coordinates of a selected image region can be used to delineate and select an image of the suspect pathology from the image data of the other modality. The suspect pathology may be highlighted in one or both of the images for quick identification and diagnosis. In step 146 diagnostic data related to the suspect pathology detected in the images is displayed to the user, as illustrated in FIGURE 9. This data may be coordinate data or diagnostic data acquired by one or both of the imaging modalities such as oxyhemoglobin measurements produced by the NIRS system and Doppler flow velocities produced by Doppler techniques in the ultrasound system. The clinician is thus provided with image regions from both imaging modalities which are related to suspect pathology, as well as diagnostic data produced by both modalities which are relevant to a diagnosis.

It should be noted that an ultrasound system and a NIRS system suitable for use in an implementation of the present invention, and in particular the component structure of the combined ultrasound/NIRS system of FIGURE 1, may be implemented in hardware, software or a combination thereof. The various embodiments and/or components of a system and its controllers, or components and controllers therein, also may be implemented as part of one or more computers or microprocessors. The computer or processor may include a computing device, an input device, a display unit and an interface, for example, for accessing the internet. The computer or processor may include a microprocessor. The microprocessor may be connected to a communication bus, for example, to access a PACS system or a data network for importing training images. The computer or processor may also include a memory. The memory devices such as the 3D memory 120 may include Random Access Memory (RAM) and/or Read Only Memory (ROM). The computer or processor further may include a storage device, which may be a hard disk drive or a removable storage drive such as a floppy disk drive, optical disk drive, solid-state drive, and the like. The storage device may also be other similar means for loading computer programs or other instructions into the computer or processor.

As used herein, the term "computer" or "module" or "processor" or "workstation" may include any processor-based or microprocessor-based system including systems using microcontrollers, reduced instruction set computers (RISC), ASICs, logic circuits, and any other circuit or processor capable of executing the functions described herein. The above examples are exemplary only and are thus not intended to limit in any way the definition and/or meaning of these terms.

The computer or processor executes a set of instructions that are stored in one or more storage elements, in order to process input data. The storage elements may also store data or other information as desired or needed. The storage elements may be in the form of an information source or a physical memory element within a processing machine. The set of instructions of an ultrasound system including those controlling the acquisition, processing, and display of ultrasound images as described above may include various commands that instruct a computer or processor as a processing machine to perform specific operations such as the registration algorithm mentioned above, as well as other methods and processes of the various embodiments of the invention. Software instructions could be used by the image processor to register the different types of image data, for instance. The set of instructions may be in the form of a software program. The software may be in various forms such as system software or application software and which may be embodied as a tangible and non-transitory computer readable medium. Numerous ultrasound and NIRS system functions are typically calculated by or under the direction of software routines. Further, the software may be in the form of a collection of separate programs or modules, or a program module within a larger program or a portion of a program module. The software also may include modular programming in the form of object-oriented programming. The processing of input data by the processing machine may be in response to operator commands, or in response to results of previous processing, or in response to a request made by another processing machine.

## Claims

1. A method comprising:
acquiring image data of a region of the body using a first imaging modality (132);
acquiring image data of the region of the body using a second imaging modality, wherein the image data of both imaging modalities is related to a common coordinate system (134);
producing images of the region of the body using the image data of each modality (136);
detecting regions of suspect pathology in the images of both modalities (138);
merging the image data of the detected regions into a 3D dataset (140);
selecting a region of suspect pathology (142);
displaying an image of one or both modalities of the selected region of suspect pathology (144); and
displaying diagnostic information related to the suspect pathology (146).

2. The method of Claim 1, wherein acquiring image data of a region of a body using a first imaging modality further comprises acquiring ultrasound image slices; and
wherein acquiring image data of the region of the body using a second imaging modality further comprises acquiring near infrared spectroscopy (NIRS) image data of a head of a subject.

3. The method of Claim 2, wherein displaying the images of both modalities of the selected region of suspect pathology further comprises registering the images of the two modalities to a template of the head to indicate an anatomical relationship of the images.

4. The method of Claim 1, wherein acquiring image data of a region of a body using a first imaging modality further comprises acquiring ultrasound image slices; and
wherein acquiring image data of the region of the body using a second imaging modality further comprises acquiring MR or CT image data of a head of a subject.

5. The method of Claim 2, wherein detecting regions of suspect pathology further comprises detecting regions of suspect pathology by processing NIRS and ultrasound images.

6. The method of Claim 5, wherein detecting regions of suspect pathology further comprises detecting regions of suspect pathology with a neural network or deep learning software.

7. The method of Claim 6, wherein detecting regions of suspect pathology with a neural network or deep learning software further comprises extracting images of both modalities of a suspect pathology for concurrent display.

8. The method of Claim 7, wherein displaying the extracted images of suspect pathology of both modalities further comprises displaying the extracted images of suspect pathology side-by-side or on top of each other.

9. The method of Claim 7, wherein displaying the extracted images of suspect pathology of both modalities further comprises displaying the extracted images in anatomical registration with an anatomical template.

10. The method of Claim 6, further comprising:
training the neural network or deep learning software with image data of suspect pathology.

11. The method of Claim 1, wherein merging the detected regions into a 3D dataset further comprises rendering together the image data of both modalities in one volumetric image.

12. The method of Claim 2, wherein acquiring image data of a region of a body using a first imaging modality further comprises acquiring ultrasound image data of a brain; and
wherein acquiring image data of the region of the body using a second imaging modality further comprises acquiring NIRS image data of the brain.

13. The method of Claim 2, wherein acquiring NIRS image data of a head of a subject further comprises acquiring NIRS image data from a plurality of NIRS emitters and sensors which are in a known spatial relation to an ultrasound transducer array.

14. A tangible, non-transitory computer readable medium comprising computer executable instructions which, when said computer executable instructions are run on a computer, cause the computer to implement the method of:
acquiring image data of a region of the body using a first imaging modality (132);
acquiring image data of the region of the body using a second imaging modality, wherein the image data of both imaging modalities is related to a common coordinate system (134);
producing images of the region of the body using the image data of each modality (136);
detecting regions of suspect pathology in the images of both modalities (138);
merging the image data of the detected regions into a 3D dataset (140);
selecting a region of suspect pathology (142);
displaying images of one or both modalities of the selected region of suspect pathology (144); and
displaying diagnostic information related to the suspect pathology(146).

15. A multimodality imaging system for diagnosing a suspect region of a body comprising:
a first imaging modality configured to acquire image data of a region of the body;
a second imaging modality configured to acquire image data of the region of the body, wherein the image data of both imaging modalities is related to a common coordinate system; and
an image processor (30) configured to:
produce images of the region of the body using the image data of each modality (136);
detect regions of suspect pathology in the images of both modalities (138);
merge the image data of the detected regions into a 3D dataset (140);
select a region of suspect pathology (142);
display images of one or both modalities of the selected region of suspect pathology (144); and
display diagnostic information related to the suspect pathology (146).

## Patentansprüche

1. Verfahren, umfassend:
Erfassen von Bilddaten eines Bereichs des Körpers unter Verwendung einer ersten bildgebenden Modalität (132);
Erfassen von Bilddaten des Bereichs des Körpers unter Verwendung einer zweiten Bildgebungsmodalität, wobei die Bilddaten beider Bildgebungsmodalitäten auf ein gemeinsames Koordinatensystem bezogen sind (134);
Erzeugen von Bildern des Bereichs des Körpers unter Verwendung der Bilddaten jeder Modalität (136);
Erkennen von Bereichen mit verdächtiger Pathologie in den Bildern beider Modalitäten (138);
Zusammenführen der Bilddaten der erkannten Bereiche zu einem 3D-Datensatz (140);
Auswählen eines Bereichs mit verdächtiger Pathologie (142);
Anzeigen eines Bildes einer oder beider Modalitäten des ausgewählten Bereichs mit verdächtiger Pathologie (144) und
Anzeigen von Diagnoseinformationen im Zusammenhang mit der verdächtigen Pathologie (146).

2. Verfahren nach Anspruch 1, wobei das Erfassen von Bilddaten eines Körperbereichs unter Verwendung einer ersten Bildgebungsmodalität weiter das Erfassen von Ultraschallbildschnitten umfasst und
wobei das Erfassen von Bilddaten des Bereichs des Körpers unter Verwendung einer zweiten Bildgebungsmodalität weiter das Erfassen von Nahinfrarotspektroskopie-(NIRS)-Bilddaten des Kopfes eines Subjekts umfasst.

3. Verfahren nach Anspruch 2, wobei das Anzeigen der Bilder beider Modalitäten des ausgewählten Bereichs mit verdächtiger Pathologie weiter das Eintragen der Bilder der beiden Modalitäten an einer Vorlage des Kopfes umfasst, um eine anatomische Beziehung der Bilder anzuzeigen.

4. Verfahren nach Anspruch 1, wobei das Erfassen von Bilddaten eines Bereichs eines Körpers unter Verwendung einer ersten Bildgebungsmodalität weiter das Erfassen von Ultraschallbildschnitten umfasst und
wobei das Erfassen von Bilddaten des Bereichs des Körpers unter Verwendung einer zweiten Bildgebungsmodalität weiter das Erfassen von MR- oder CT-Bilddaten des Kopfes eines Subjekts umfasst.

5. Verfahren nach Anspruch 2, wobei das Erkennen von Bereichen mit verdächtiger Pathologie weiter das Erkennen von Bereichen mit verdächtiger Pathologie durch Verarbeiten von NIRS- und Ultraschallbildern umfasst.

6. Verfahren nach Anspruch 5, wobei das Erkennen von Bereichen mit verdächtiger Pathologie weiter das Erkennen von Bereichen mit verdächtiger Pathologie mit einem neuronalen Netz oder Deep-Learning-Software umfasst.

7. Verfahren nach Anspruch 6, wobei das Erkennen von Bereichen mit verdächtiger Pathologie mit einem neuronalen Netz oder Deep-Learning-Software weiter das Extrahieren von Bildern beider Modalitäten mit einer verdächtigen Pathologie zur gleichzeitigen Anzeige umfasst.

8. Verfahren nach Anspruch 7, wobei das Anzeigen der extrahierten Bilder mit verdächtiger Pathologie beider Modalitäten weiter das Anzeigen der extrahierten Bilder mit verdächtiger Pathologie nebeneinander oder übereinander umfasst.

9. Verfahren nach Anspruch 7, wobei das Anzeigen der extrahierten Bilder mit verdächtiger Pathologie beider Modalitäten weiter das Anzeigen der extrahierten Bilder in anatomischer Eintragung mit einer anatomischen Vorlage umfasst.

10. Verfahren nach Anspruch 6, weiter umfassend:
Trainieren des neuronalen Netzes oder der Deep-Learning-Software mit Bilddaten mit verdächtiger Pathologie.

11. Verfahren nach Anspruch 1, wobei das Zusammenführen der erkannten Bereiche zu einem 3D-Datensatz weiter das Zusammenführen der Bilddaten beider Modalitäten in einem volumetrischen Bild umfasst.

12. Verfahren nach Anspruch 2, wobei das Erfassen von Bilddaten eines Bereichs eines Körpers unter Verwendung einer ersten Bildgebungsmodalität weiter das Erfassen von Ultraschallbilddaten des Gehirns umfasst und
wobei das Erfassen von Bilddaten des Bereichs des Körpers unter Verwendung einer zweiten Bildgebungsmodalität weiter das Erfassen von NIRS-Bilddaten des Gehirns umfasst.

13. Verfahren nach Anspruch 2, wobei das Erfassen von NIRS-Bilddaten eines Kopfes eines Subjekts weiter das Erfassen von NIRS-Bilddaten von einer Vielzahl von NIRS-Emittern und -Sensoren umfasst, die sich in einer bekannten räumlichen Beziehung zu einem Ultraschallwandlerarray befinden.

14. Materielles, nichtflüchtiges computerlesbares Medium, das computerausführbare Anweisungen umfasst, die, wenn die computerausführbaren Anweisungen auf einem Computer ausgeführt werden, den Computer veranlassen, das folgende Verfahren zu implementieren:
Erfassen von Bilddaten eines Bereichs des Körpers unter Verwendung einer ersten Bildgebungsmodalität (132);
Erfassen von Bilddaten des Bereichs des Körpers unter Verwendung einer zweiten Bildgebungsmodalität, wobei die Bilddaten beider Bildgebungsmodalitäten auf ein gemeinsames Koordinatensystem bezogen sind (134);
Erzeugen von Bildern des Bereichs des Körpers unter Verwendung der Bilddaten jeder Modalität (136);
Erkennen von Bereichen mit verdächtiger Pathologie in den Bildern beider Modalitäten (138);
Zusammenführen der Bilddaten der erkannten Bereiche zu einem 3D-Datensatz (140);
Auswählen eines Bereichs mit verdächtiger Pathologie (142);
Anzeigen von Bildern einer oder beider Modalitäten des ausgewählten Bereichs mit verdächtiger Pathologie (144); und
Anzeigen von Diagnoseinformationen im Zusammenhang mit der verdächtigen Pathologie (146).

15. Multimodales Bildgebungssystem zum Diagnostizieren eines verdächtigen Bereichs eines Körpers, umfassend:
eine erste Bildgebungsmodalität, die so konfiguriert ist, dass sie Bilddaten eines Bereichs des Körpers erfasst;
eine zweite Bildgebungsmodalität, die so konfiguriert ist, dass sie Bilddaten des Bereichs des Körpers erfasst, wobei die Bilddaten beider Bildgebungsmodalitäten auf ein gemeinsames Koordinatensystem bezogen sind; und
einen Bildprozessor (30), der zu Folgendem konfiguriert ist:
Bilder des Bereichs des Körpers unter Verwendung der Bilddaten jeder Modalität zu erzeugen (136);
Bereiche mit verdächtiger Pathologie in den Bildern beider Modalitäten zu erkennen (138);
die Bilddaten der erkannten Bereiche zu einem 3D-Datensatz zusammenzuführen (140);
einen Bereich mit verdächtiger Pathologie auszuwählen (142);
Bilder einer oder beider Modalitäten des ausgewählten Bereichs mit verdächtiger Pathologie anzuzeigen (144) und
Diagnoseinformationen im Zusammenhang mit der verdächtigen Pathologie anzuzeigen (146).

## Revendications

1. Procédé comprenant :
l'acquisition de données d'image d'une région du corps à l'aide d'une première modalité d'imagerie (132) ;
l'acquisition de données d'image de la région du corps à l'aide d'une deuxième modalité d'imagerie, dans lequel les données d'image des deux modalités d'imagerie sont liées à un système de coordonnées commun (134) ;
la production d'images de la région du corps en utilisant les données d'image de chaque modalité (136) ;
la détection des régions de pathologie suspecte dans les images des deux modalités (138) ;
la fusion des données d'image des régions détectées dans un ensemble de données 3D (140) ;
la sélection d'une région de pathologie suspecte (142) ;
l'affichage d'une image de l'une et/ou l'autre des modalités de la région sélectionnée de la pathologie suspecte (144) ; et
l'affichage d'informations diagnostiques relatives à la pathologie suspectée (146).

2. Procédé selon la revendication 1, dans lequel l'acquisition de données d'image d'une région du corps à l'aide d'une première modalité d'imagerie comprend en outre l'acquisition de coupes d'images ultrasonores ; et
dans lequel l'acquisition de données d'image de la région du corps à l'aide d'une deuxième modalité d'imagerie comprend en outre l'acquisition de données d'image de spectroscopie proche infrarouge (NIRS) de la tête d'un sujet.

3. Procédé selon la revendication 2, dans lequel l'affichage des images des deux modalités de la région sélectionnée de pathologie suspecte comprend en outre l'enregistrement des images des deux modalités sur un modèle de la tête pour indiquer une relation anatomique des images.

4. Procédé selon la revendication 1, dans lequel l'acquisition de données d'image d'une région du corps à l'aide d'une première modalité d'imagerie comprend en outre l'acquisition de coupes d'images ultrasonores ; et
dans lequel l'acquisition de données d'image de la région du corps à l'aide d'une deuxième modalité d'imagerie comprend en outre l'acquisition de données d'image IRM ou CT de la tête d'un sujet.

5. Procédé selon la revendication 2, dans lequel la détection des régions de pathologie suspecte comprend en outre la détection des régions de pathologie suspecte par traitement d'images NIRS et d'échographie.

6. Procédé selon la revendication 5, dans lequel la détection des régions de pathologie suspecte comprend en outre la détection des régions de pathologie suspecte à l'aide d'un réseau neuronal ou d'un logiciel d'apprentissage profond.

7. Procédé selon la revendication 6, dans lequel la détection de régions de pathologie suspecte à l'aide d'un réseau neuronal ou d'un logiciel d'apprentissage profond comprend en outre l'extraction d'images des deux modalités d'une pathologie suspecte pour un affichage simultané.

8. Procédé selon la revendication 7, dans lequel l'affichage des images extraites de la pathologie suspecte des deux modalités comprend en outre l'affichage des images extraites de la pathologie suspecte côte à côte ou l'une sur l'autre.

9. Procédé selon la revendication 7, dans lequel l'affichage des images extraites de la pathologie suspecte des deux modalités comprend en outre l'affichage des images extraites par alignement anatomique avec un modèle anatomique.

10. Procédé selon la revendication 6, comprenant en outre :
la formation du réseau neuronal ou du logiciel d'apprentissage profond avec des données d'images de pathologies suspectes.

11. Procédé selon la revendication 1, dans lequel la fusion des régions détectées dans un ensemble de données 3D comprend en outre le rendu conjoint des données d'image des deux modalités dans une seule image volumétrique.

12. Procédé selon la revendication 2, dans lequel l'acquisition de données d'image d'une région du corps au moyen d'une première modalité d'imagerie comprend en outre l'acquisition de données d'image ultrasonore du cerveau ; et
dans lequel l'acquisition de données d'image de la région du corps à l'aide d'une deuxième modalité d'imagerie comprend en outre l'acquisition de données d'image NIRS du cerveau.

13. Procédé selon la revendication 2, dans lequel l'acquisition de données d'image NIRS de la tête d'un sujet comprend en outre l'acquisition de données d'image NIRS à partir d'une pluralité d'émetteurs et de capteurs NIRS qui sont dans une relation spatiale connue avec un réseau de transducteurs à ultrasons.

14. Support tangible, non transitoire et lisible par ordinateur, comprenant des instructions exécutables qui, lorsqu'elles sont exécutées sur un ordinateur, amènent l'ordinateur à mettre en œuvre le procédé suivant :
l'acquisition de données d'image d'une région du corps à l'aide d'une première modalité d'imagerie (132) ;
l'acquisition de données d'image de la région du corps à l'aide d'une deuxième modalité d'imagerie, dans laquelle les données d'image des deux modalités d'imagerie sont liées à un système de coordonnées commun (134) ;
la production d'images de la région du corps en utilisant les données d'image de chaque modalité (136) ;
la détection de régions de pathologie suspecte dans les images des deux modalités (138) ;
la fusion des données d'image des régions détectées dans un ensemble de données 3D (140) ;
la sélection d'une région de pathologie suspecte (142) ;
l'affichage d'images de l'une et/ou l'autre des modalités de la région sélectionnée de pathologie suspecte (144) ; et
l'affichage d'informations diagnostiques relatives à la pathologie suspectée (146).

15. Système d'imagerie multimodale pour le diagnostic d'une région suspecte du corps comprenant :
une première modalité d'imagerie configurée pour acquérir des données d'image d'une région du corps ;
une deuxième modalité d'imagerie configurée pour acquérir des données d'image de la région du corps, dans lequel les données d'image des deux modalités d'imagerie sont liées à un système de coordonnées commun ; et
un processeur d'images (30) configuré pour :
produire des images de la région du corps en utilisant les données d'image de chaque modalité (136) ;
détecter les régions de pathologie suspecte dans les images des deux modalités (138) ;
fusionner les données d'image des régions détectées dans un ensemble de données 3D (140) ;
sélectionner une région de pathologie suspecte (142) ;
afficher des images de l'une et/ou l'autre des modalités de la région sélectionnée de la pathologie suspecte (144) ; et
afficher les informations diagnostiques relatives à la pathologie suspectée (146).
